# EUROPÄISCHE PATENTSCHRIFT

(11) **EP 1 042 991 B1**
(45) Veröffentlichungstag und Bekanntmachung des Hinweises auf die Patenterteilung: **13.04.2005**
(21) Anmeldenummer: 99122684.6
(22) Anmeldetag: 15.11.1999
(51) Int. Cl.: A61B 19/00, A61F 11/00, H04R 25/00

(54) **Implantierbares Positionier- und Fixiersystem für aktorische und sensorische Implantate**
Implantable positioning and fixation system for actoric and sensoric implants
Système de positionnement et fixation implantable pour implants actoriques et sensoriels

(30) Priorität: 07.04.1999 DE 19915684
(43) Veröffentlichungstag der Anmeldung: 11.10.2000
(73) Patentinhaber: PHONAK AG, 8712 Stäfa (CH)
(72) Erfinder: Müller, Gerd Dr., 85716 Lohhof (DE)
(74) Vertreter: Schwan, Gerhard, Dipl.-Ing.

(56) Entgegenhaltungen:
- DE-A- 19 618 964
- GB-A- 2 239 605
- US-A- 5 601 551
- US-A- 5 776 144
- US-A- 5 853 223

## Beschreibung

Die Erfindung betrifft eine dauerhaft implantierbare Vorrichtung für die intraoperative Positionierung und nachfolgende Fixierung von implantierbaren aktiven oder passiven, aktorischen oder sensorischen Mitteln (im folgenden kurz als Mittel bezeichnet) im menschlichen Körper, insbesondere im Mastoid- und Mittelohrbereich des Schädels.

Angesichts der außerordentlich kleinen und empfindlichen anatomischen Strukturen im menschlichen Körper, insbesondere im Mastoid- und Mittelohrbereich des Schädels, ist das längere (mehr als wenige Sekunden) dauernde, handgeführte Beibehalten der Position eines Mittels nahezu unmöglich oder erfordert vom Operateur einen erheblichen Kraftund Konzentrationsaufwand. Viele Eingriffe im Körper, vor allem im Schädelbereich, erfordern aber gerade eine über längere Zeiträume fixierbare, zielpunktgerichtete Positionierung geeigneter aktorischer bzw. sensorischer Mittel.

Aufgrund der Tatsache, daß handgeführte aktorische oder sensorische Mittel für mikrochirurgische, therapeutische oder diagnostische Manipulationen an empfindlichen Kleinstrukturen z.B. des Schädels stets das Risiko beinhalten, daß aufgrund der möglichen Relativbewegungen zwischen handgeführtem Mittel und dem Körper des Patienten diese Zielstrukturen unter Umständen beschädigt oder verändert werden, besteht in der Technik schon seit längerem der Wunsch, ein Positioniersystem zur Hand zu haben, welches sich am Körper, insbesondere am Schädel, mittels einer Halterung ortsfest verankern läßt.

Aus dem Stand der Technik sind verschiedene implantierbare Aktorhalterungen bekannt. Eine Halterung als Bestandteil eines teilimplantierbaren piezoelektrischen Hörgerätekonzepts zur Stimulation des Steigbügels wurde von N. Yanagihara, K. Gyo und Y. Hinohira in dem in "Otolaryngologic Clinics Of North America" erschienenen Artikel "Partially Implantable Hearing Aid Using Piezoelectric Ceramic Ossicular Vibrator", Vol. 28, No.1, Februar 1995, Seiten 85-97, vorgestellt. Der externe Geräteteil ist wie ein konventionelles, hinter dem Ohr zu tragendes Hörgerät ausgeführt und beinhaltet Mikrophon, Verstärker, Batterie und die externe Sendespule. Der interne, auf der Schädelkalotte fixierte Geräteteil dient zur Aufnahme der inneren Empfangsspule. Zur Positionierung und Fixierung des piezoelektrischen Bimorph-Wandlers im Mittelohr ist ein relativ einfaches L-förmiges knochenverankertes Befestigungselement vorgesehen. Es handelt sich dabei um ein auf der Schädelkalotte mit zwei Knochenschrauben fixierbares Halteblech, das sich aus einer Metallplatte mit zwei Langlöchern und einer senkrecht daran befestigten Drahtachse zusammensetzt. Nach Aufschrauben der Metallplatte auf die Schädelkalotte zeigt die Drahtachse in das Mittelohr (nach medial). Auf der Drahtachse läßt sich eine Hülse axial verschieben und somit der wiederum an der Hülse befestigte piezoelektrische Bimorph-Wandler positionieren. Das ermöglicht einen axialen und einen rotatorischen Freiheitsgrad auf der Drahtachse. Nach Abbau von Hammer und Amboß kann das freie Ende des Piezoelementes vorzugsweise direkt am Steigbügelkopf mit Cyanacrylatkleber befestigt werden.

Ein weiteres Haltesystem für ein gehörverbesserndes aktorisches Implantatmodul wurde von J. Frederickson, J.M. Coticchia und S. Khosla in dem ebenfalls in "Otolaryngologic Clinics Of North America" erschienenen Artikel mit dem Titel "Ongoing Investigations Into An Implantable Electromagnetic Hearing Aid For Moderate To Severe Sensorineural Hearing Loss", Vol. 28, No.1., Februar 1995, Seiten 107-119, beschrieben. Es ist Bestandteil eines bisher im Tiermodell erprobten, teilimplantierbaren elektromagnetischen Hörgeräts, bei dessen Implantation mit einem chirurgischen Laser ein kleines Loch in den Amboßkörper zur Befestigung eines Permanentmagneten eingebracht wird. Der Laserkopf wird dabei in einer in den Mastoidknochen eingeschraubten Gewindehülse mit Innen- und Außengewinde geführt, deren Längsachse auf den Amboßkörper zeigt. Nach Setzen der Laserbohrung am Amboß und Entnahme des Laserkopfs kann in diese Gewindehülse der elektromagnetische Antrieb ("transducer probe tip") eingeschraubt und nach medial zum ossikelfesten Magneten positioniert werden.

Ein anderes Haltesystem entwickelten Maniglia et al. für einen teilimplantierbaren elektromagnetischen Mittelohrstimulator [A.J. Maniglia, W.H. Ko, M. Rosenbaum, T. Falk, W.L. Zhu, N.W. Frenz, J. Werning, J. Masin, A. Stein und A. Sabri, "Contactless Semi-Implantable Electromagnetic Middle Ear Device For The Treatment Of Sensorineural Hearing Loss", erschienen in "Otolaryngologic Clinics Of North America", 1995, Vol. 28, No. 1, February 1995, Seiten 121 und folgende]. Dabei wird ein kleiner Magnet mittels chirurgischem Zement auf den Amboß geklebt. Entlang eines aus Titan gefertigten Führungsschaftes, der sich in das Mastoid implantieren läßt, kann die Antriebsspule bis auf einen Luftspalt von maximal 1 mm zum ossikelfesten Permanentmagneten positioniert werden. Dieser Titanschaft besitzt wie in der Ausführung von Yanagihara et al. zwei Langlöcher und ein zusätzliches Bohrloch zur Fixierung mittels dreier Knochenschrauben auf der Schädelkalotte. Mittels einer Gewindeachse lassen sich ein Elektronikmodul und die daran befestigte Antriebsspule nach medial in einer Langlochführung positionieren und über eine Schraube mit Kontermutter auf dem Schaft fixieren.

Die vorstehend skizzierten bekannten Haltesysteme dienen zur dauerhaften Fixierung von Hörgerätebauteilen auf dem Schädelknochen bzw. in der Nähe des Mittel- und Innenohres. Sie weisen insgesamt eine äußerst eingeschränkte intraoperative Positionierbarkeit aufgrund fehlender nutzbarer Freiheitsgrade aus, und sie müssen allesamt durch mehr oder weniger präzises manuelles Zurechtbiegen an die anatomischen Gegebenheiten des Implantationsortes sowie an die vorgefundene Lage des Zielortes im Mittelohr angepaßt werden. Das erste sowie das dritte der beschriebenen Haltesysteme benötigen zudem Kleber bzw. chirurgischen Zement zur Bauteilfixierung, die sich häufig aufgrund von Festigkeitsverlusten nicht als Langzeitimplantate eignen.

Eine implantierbares fixierbares Positioniersystem für die feste, spielfreie Anbindung an den menschlichen Körper, insbesondere an den menschlichen Schädel, das ohne Klebstoffe oder chirurgische Zemente und ohne manuelles Zurechtbiegen des Implantathalters dauerhaft befestigt werden kann, um frei von Relativbewegungen chirurgische, therapeutische oder diagnostische Sensoren bzw. Aktoren im Körper zu positionieren und in der gefundenen Position fest zu fixieren, ist aus US-PS 5 788 711 bekannt, welches dem ersten Teil des Anspruchs 1 entspricht. Diese System ist versehen mit einer an dem menschlichen Körper fixierbaren Halterung; einem an der Halterung angebrachten, mit einem Hilfswerkzeug manuell positionierbaren und durch einen Klemmechanismus fixierbaren Kugelgelenk; einer mit der Gelenkkugel des Kugelgelenks fest verbundenen Führungsschiene; einer in der Gelenkkugel und der Führungsschiene drehbar, aber axial unverschiebbar gelagerten Gewindespindel; einem in der Führungsschiene und auf der Gewindespindel für eine axiale Verstellbewegung geführten Schlitten, der eine mit der Gewindespindel in Gewindeeingriff stehende, gegen eine Drehbewegung relativ zu der Führungsschiene gesicherte Spindelmutter aufweist und der durch Drehen der Gewindespindel mittels eines Hilfswerkzeugs entlang der Führungsschiene frei positionierbar ist; und einer an dem Schlitten angebrachten Aufnahme für ein zu positionierendes bzw. fixierendes aktorisches oder sensorisches Mittel.

Das letztgenannte Positioniersystem hat sich in der Praxis bei der Implantation von Hörhilfen bereits gut bewährt. Gleichwohl sind weitere Verbesserungen insbesondere hinsichtlich der Führungsgenauigkeit und Leichtgängigkeit erwünscht.

Diese Aufgabe wird gelöst durch ein dauerhaft implantierbares fixierbares Positioniersystem für die feste, spielfreie Anbindung an einem Knochen des menschlichen Körpers, insbesondere dem Schädelknochen, mit:
- einem eine Gelenkkugel und eine Kugelaufnahme aufweisenden Kugelgelenk, dessen Gelenkkugel mit Bezug auf die Kugelaufnahme mittels eines Hilfswerkzeugs manuell schwenkbar und durch einen Klemmechanismus fixierbar ist,
- einer mit der Gelenkkugel des Kugelgelenks fest verbundenen geraden Führungsschiene,
- einer mit Bezug auf die Führungsschiene drehbar, aber in Axialrichtung unverschiebbar gelagerten, Außengewinde aufweisenden Gewindespindel,
- einem Schlitten, der eine Spindelmutter mit einem mit dem Außengewinde der Gewindespindel in Gewindeeingriff stehenden Innengewinde aufweist und der durch manuelles Drehen der Gewindespindel mit einem Hilfswerkzeug zwischen Endanschlägen entlang der Führungsschiene frei positionierbar ist,
- einer an dem Schlitten angebrachten Aufnahme für ein zu positionierendes beziehungsweise fixierendes aktorisches oder sensorisches Mittel, und
- einem an dem Knochen anschraubbaren Halteteil, an dem die Kugelaufnahme und der Klemmechanismus des Kugelgelenks angebracht sind,
das erfindungsgemäß dadurch gekennzeichnet ist, daß
die Führungsschiene Führungs-Außenflächen aufweist, die in Gleiteingriff mit Führungs-Innenflächen des Schlittens gehalten sind, und die Führungs-Innenflächen von Wangen des Schlittens gebildet sind, die mit Bezug auf die Spindelmutter in einer zur Längsachse der Gewindespindel senkrecht stehenden Ebene federnd angeordnet sind.

Bei dem Positioniersystem nach der Erfindung lassen sich - bei vorgegebenen Gesamtabmessungen des Systems - relativ großflächige Anlageflächen zwischen Führungsschiene und Schlitten erreichen. Dem kommt praktische Bedeutung vor allem dann zu, wenn die Gesamtabmessungen des Systems sehr klein sein müssen, wie dies typischerweise bei im menschlichen Körper zu implantierenden Geräten, beispielsweise Hörhilfen, der Fall ist. Eine relativ großflächige Anlage zwischen Führungsschiene und Schlitten hat vergleichsweise niedrige Flächenpressungen zur Folge. Die dadurch erzielbare Verringerung der Reibungsanteile sorgt für hohe Leichtgängigkeit über den gesamten Verfahrweg des Schlittens. Das System ist gegenüber Querkräften in axialer, radialer und angularer Richtung besonders stabil.

Das körperfest zu fixierende Positioniersystem dient mit seiner Aufnahme für beliebige aktive oder passive, aktorische, sensorische, mechanische oder optische Mittel als "künstliche, tremorfreie Hand" des Chirurgen, um das freie Wirkende des Mittels zu einem körperfesten Zielpunkt zu positionieren und dann zu fixieren, ohne daß dabei nennenswerte risikobehaftete Relativbewegungen auftreten.

Die Führungs-Innenflächen sind von Wangen des Schlittens gebildet, die mit Bezug auf die Spindelmutter in einer zur Längsachse der Gewindespindel senkrecht stehenden Ebene federnd angeordnet sind. Dadurch läßt sich ein zwangsweises gegenseitiges Andrücken der Führungsflächen von Schlitten und Führungsschiene erreichen. Aufgrund der federnden Wangen werden unvermeidbare Fertigungstoleranzen selbsttätig ausgeglichen. Es läßt eine spielfreie Führung sowohl in radialer als auch in angularer Richtung sichergestellen. Ein Zusammenbau von Schlitten und Führungsschiene unter Vorspannung ist möglich. Die wirksame Vorspannkraft kann dabei durch entsprechende Wahl von Stärke und/oder Länge der federnden Wangen eingestellt werden.

Bevorzugte Ausführungsformen der Erfindung sind in der Unteranspüchen beschrieben.

Eine besonders kompakte und robuste Anordnung wird dabei erhalten, wenn die Wangen an Federarmen angeformt sind, die mit einem die Spindelmutter aufweisenden Schlittenkörper einstückig verbunden sind.

In weiterer Ausgestaltung der Erfindung liegen die Führungs-Innenflächen des Schlittens und die Führungs-Außenflächen der Führungsschiene jeweils einander diametral gegenüber und sind spiegelsymmetrisch zu einer die Längsachse der Spindelmutter enthaltenden Längssymmetrieebene des Schlittens beziehungsweise spiegelsymmetrisch zu einer die Längsachse der Gewindespindel enthaltenden Längssymmetrieebene der aus Führungsschiene; und Gewindespindel bestehenden Baugruppe angeordnet, wobei die Führungs-Innenflächen des Schlittens und die Führungs-Außenflächen der Führungsschiene mit der Längssymmetrieebene des Schlittens beziehungsweise der Längssymmetrieebene der aus Führungsschiene und Gewindespindel bestehenden Baugruppe jeweils einen Winkel einschließen, beispielsweise einen Winkel im Bereich von 10° bis 60°. Die Schrägstellung der Führungsflächen bewirkt eine selbsttätige Zentrierung des Schlittens auf der Führungsschiene. Die Gewindespindel selbst braucht keine Führungsfunktion mehr zu erfüllen; sie ist dann nur noch für den Vortrieb des Schlittens zuständig. Insgesamt wird eine Leichtgängigkeit bei gleichzeitiger hoher Führungsgenauigkeit erreicht.

Die Spindelmutter und die Gewindespindel sind vorteilhaft mit selbsthemmendem Gewinde versehen, das heißt einem Gewinde, dessen Steigung so bemessen ist, daß zwar durch Drehen der Spindel ein Verstellen des Schlittens bewirkt werden kann, aber nicht umgekehrt durch Verstellen des Schlittens ein Drehen der Spindel. Auf diese Weise wird ein unbeabsichtigtes Verstellen des Schlittens und des mit ihm verbundenen aktorischen oder sensorischen Mittels sicher und auf einfache Weise verhindert.

Zweckmäßig ist die Gewindespindel an ihrem kugelseitigen Ende und an ihrem von der Gelenkkugel abliegenden Ende in der die Gelenkkugel und die Führungsschiene umfassenden Anordnung drehbar gelagert.

Vorteilhaft weist die Gelenkkugel eine Aufnahme zum Ansetzen des dem Schwenken der Gelenkkugel dienenden Hilfswerkzeugs auf, während die Gewindespindel an ihrem der Gelenkkugel zugewendeten Ende mit einer Aufnahme zum Ansetzen des Hilfswerkzeugs für das Drehen der Spindel aufweist. Dabei ist die Anordnung vorzugsweise so getroffen, daß die genannten Aufnahmen zueinander koaxial angeordnet sind und die Aufnahme zum Ansetzen des dem Drehen der Gewindespindel dienenden Hilfswerkzeugs durch die Aufnahme zum Ansetzen des dem Schwenken der Gelenkkugel dienenden Hilfswerkzeugs hindurch zugänglich ist.

Um die Teileanzahl klein zu halten und die Handhabung besonders einfach zu gestalten, ist zum Betätigen des Klemmechanismus zweckmäßig ein einziges Stellelement vorgesehen.

Der Klemmechanismus kann auf unterschiedliche Weise aufgebaut sein, unter anderem als Keil-Klemmvorrichtung, die eine auf einer schrägen Ebene gleitende Druckkalotte aufweist, die durch Anziehen einer das Stellelement bildenden Klemmschraube gegen die Gelenkkugel anpreßbar ist.

Entsprechend einer abgewandelten Ausführungsform kann der Klemmechanismus als Ring-Klemmvorrichtung ausgebildet sein, der zweckmäßig einen in ein Gewinde der Kugelaufnahme einschraubbaren und gegen die Gelenkkugel anpreßbaren Klemmring aufweist, der das Stellelement bildet.

Der Klemmechanismus kann aber auch als Hebel-Klemmvorrichtung mit einer als Hebel wirkenden Druckkalotte ausgebildet sein, die durch Anziehen einer das Stellelement bildenden Klemmschraube um ein Hebel-Gegenlager schwenkbar und gegen die Gelenkkugel anpreßbar ist.

Eine weitere geeignete Ausführungsform des Klemmechanismus ist eine Exzenter-Klemmvorrichtung, die zweckmäßig eine Druckkalotte aufweist, die mit einer Exzenterscheibe zusammenwirkt, die ihrerseits mit einer das Stellelement bildenden Klemmschraube drehfest verbundenen ist, wobei die Druckkalotte durch Drehen der Klemmschraube und damit der Exzenterscheibe gegen die Gelenkkugel anpreßbar ist.

Als Klemmechanismus kann ferner eine Schellen-Klemmvorrichtung vorgesehen sein, wobei die Kugelaufnahme derart geschlitzt sein kann, daß sie die Gelenkkugel federnd umschließt, und wobei die Kugelaufnahme durch Anziehen einer das Stellelement bildenden Klemmschraube gegen die Gelenkkugel anpreßbar ist.

Der Klemmechanismus kann auch als Druckstift- Klemmvorrichtung ausgebildet sein. Dabei kann ein das Stellelement bildender Gewinde-Druckstift in eine Gewindebohrung des Halteteils eingeschraubt und durch Drehen gegen die Gelenkkugel anpreßbar sein. Entsprechend einer abgewandelten Ausführungsform kann ein das Stellelement bildender Druckstift durch Verschwenken eines Exzenters gegen die Gelenkkugel anpreßbar und zum Vorfixieren der Gelenkkugel in Richtung auf die Gelenkkugel federnd vorgespannt sein.

Das Stellelement ist vorzugsweise mit einem Verlierschutz versehen, der auch ein unbeabsichtigtes Herausfallen der Gelenkkugel aus der Kugelaufnahme verhindert.

Das dem Drehen der Gewindespindel dienende Hilfswerkzeug hat zweckmäßig einen Kugelkopf mit Mehrkantprofil, der mit einer ein komplementäres Mehrkantprofil aufweisenden Ausnehmung der Gewindespindel nach Art eines Gleichlaufgelenks (Torx®) in Formschluß-Eingriff bringbar ist. Dadurch läßt sich die Gewindespindel auch dann sicher Drehen, wenn es die Platzverhältnisse am Implantationsort nicht erlauben, die Achse des zweiten Hilfswerkzeugs mit der Achse der Gewindespindel auszurichten.

Das Positioniersystem kann grundsätzlich aus beliebigen biokompatiblen Werstoffen aufgebaut sein. Vorzugsweise ist es jedoch aus implantierbaren Metallen, wie z.B. Reintitan, seinen implantierfähigen Legierungen sowie Implantat-Edelstählen, gefertigt.

Die aktorischen oder sensorischen Mittel können zur Diagnose, Therapie und/oder für chirurgische Applikationen für die temporäre oder dauerhafte Implantation der genannten Mittel ausgelegt sein. Bei dem aktorischen Mittel kann es insbesondere um einen implantierbaren elektromechanischen Hörgerätewandler handeln, wie er unter anderem in der US-PS 5 277 694 beschrieben ist.

Das Positioniersystem kann zum Positionieren und Ankoppeln eines daran befestigten Hörgerätewandlers zu jedem beliebigen Punkt des Mittelohres einschließlich jeder beliebigen Ankoppelstelle an das Innenohr, wie einem natürlichen oder artifiziellen Fenster, als körperfestem Zielpunkt und zum Fixieren des Hörgerätewandlers in dieser Lage ausgelegt sein. Bei dem Zielpunkt kann es insbesondere um jeden beliebigen Punkt von Hammer, Amboß oder Steigbügel handeln. Der Hörgerätewandler kann als aktorische Komponente eines teilweise oder vollständig implantierbaren Hörgerätes vorgesehen sein.

Das Positioniersystem kann aufgrund der Freiheitsgrade des Kugelgelenks und der aus Spindelmutter und Gewindespindel bestehenden Anordnung zusammengefaßt für eine axiale und drei rotatorische Bewegungen des aktorischen oder sensorischen Mittels und seines freien Wirkendes in Körperöffnungen des menschlichen Körpers ausgelegt und insbesondere so aufgebaut sein, daß bei gelöster Klemmung des Kugelgelenks eine Verstellung aller drei rotatorischen Freiheitsgrade desselben mittels des zugehörigen Hilfswerkzeugs erfolgen kann.

Die momentane Position der drei rotatorischen Freiheitsgrade bei gelöster Klemmung des Kugelgelenks wird vorzugsweise durch Reibkräfte gesichert und nach dem Schließen der Klemmung dauerhaft beibehalten.

Um dem behandelnden Operateur das Einbringen und Einstellen des Positioniersystems zu erleichtern, zeigen die Bedienteile zur manuellen Positionierung des Kugelgelenks und des Schlittens sowie zur Klemmung des Kugelgelenks vorzugsweise vom Körper des Patienten weg zum Operateur. In dieser Hinsicht erweist es sich außerdem als zweckmäßig, wenn die Konstruktion und die geometrischen Abmessungen des Positioniersystems derart gestaltet sind, daß der bedienende Operateur beim Arbeiten mit bloßem Auge oder bei Verwendung eines Mikroskopes stets freie Sicht auf mindestens das freie Wirkende des aktorischen oder sensorischen Mittels sowie auf den Implantationsbereich samt Zielpunkt im Körper des Patienten behält. Auf diese Weise werden die sonst durch eine mögliche Fehlpositionierung des Mittels verursachten Risiken für den Patienten besonders gering gehalten.

Als bevorzugte Körperregion, in die das Positioniersystem samt aktorischem oder sensorischem Mittel eingebracht werden kann, kommt insbesondere eine unter der Ohrmuschel im Schädelknochen liegende Mastoidhöhle in Frage. Sie läßt sich durch mikrochirurgische Standardtechniken eröffnen. Ihr Volumen beträgt dann einige Kubikzentimeter, und dieses Volumen ist großen patientenindividuellen, räumlichen Exemplarstreuungen unterworfen.

Das Halteteil des Positioniersystems wird in diesem Fall auf die Oberfläche des unmittelbar an den Rand der geschaffenen Mastoidhöhle angrenzenden Schädelknochens aufgeschraubt. Das System ist so konzipiert, daß es das Niveau der Kalottenwölbung nicht überragt. Somit ist gewährleistet, daß sich das implantierte System nach der Operation nicht unter der Haut abzeichnet.

Mittels des vorliegenden Positioniersystems ist die tremorfreie intraoperative Positionierung und Fixierung eines beliebigen aktorischen oder sensorischen Mittels zum Beispiel an einem der drei Ossikel der Gehörknöchelchenkette (Hammer, Amboß, Steigbügel), der knöchernen Trennwand zwischen luftgefülltem Mittelohr und flüssigkeitsgefülltem Innenohr (Promontorium), im flüssigkeitsgefüllten Innenohr selbst sowie dem angrenzenden Vestibularorgan möglich. Weitere Anwendungen des erfindungsgemäßen Systems liegen in der kurzzeitigen, intraoperativen Laserchirurgie im gesamten Schädelbereich einschließlich Mikrokoagulationen oder Gewebeverödungen. Bei Einkopplung eines Meßlasers können intraoperativ Schwingungen z.B. der Gehörknöchelchenkette, des Trommelfells oder der Rundfenstermembran berührungsfrei gemessen werden.

Bei einer bevorzugten Ausführungsform der Erfindung wird das oben beschriebene implantierbare Positioniersystem mit einem aktorischen Hörgerätewandler kombiniert, der zur gehörverbessernden vibratorischen Stimulation von Schwerhörigen dient. Diese bevorzugte Ausführung ist somit Bestandteil einer teilweise oder vollständig implantierbaren Hörhilfe.

Bevorzugte Ausführungsbeispiele der Erfindung sind nachfolgend unter Bezugnahme auf die beiliegenden Zeichnungen näher erläutert. Es zeigen :
- Fig. 1: eine Schnittansicht einer bevorzugten Ausführungsform des Positioniersystems mit darin gehaltenem Hörgeräteaktor nach Implantation in einer Mastoidhöhle des menschlichen Schädels, wobei das freie Wirkende des Hörgeräteaktors durch die hintere Gehörgangswand zum Zielpunkt im Mittelohr zeigt;
- Fig. 2: eine perspektivische Teilansicht des in Fig. 1 gezeigten Positioniersystems;
- Fig. 3: eine Schnittansicht eines Teils des Positioniersystems gemäß den Fign. 1 und 2;
- Fig. 4: einen Querschnitt entlang der Linie IV-IV in Fig. 3;
- Fig. 5: eine perspektivische Ansicht von Gelenkkugel und Führungsschiene des Positioniersystems gemäß den Fign. 1 bis 4;
- Fig. 6: einen Längsschnitt des Halteteils und der Kugelaufnahme des Positioniersystems gemäß den Fign. 1 bis 5;
- Fign. 7 bis 14: verschiedene Ausführungsbeispiele des Kugelgelenk-Klemmechanismus, teils im Schnitt und teils in Draufsicht;
- Fig. 15: eine Schnittansicht eines Teils der Gelenkkugel, der Gewindespindel und eines Ausführungsbeispiels des zum Drehen der Gewindespindel vorgesehenen Hilfswerkzeuges und
- Fign. 17 bis 21: weitere Ausführungsbeispiele des Kugelgelenk-Klemmechanismus, teils im Schnitt und teils in perspektivischer Darstellung.

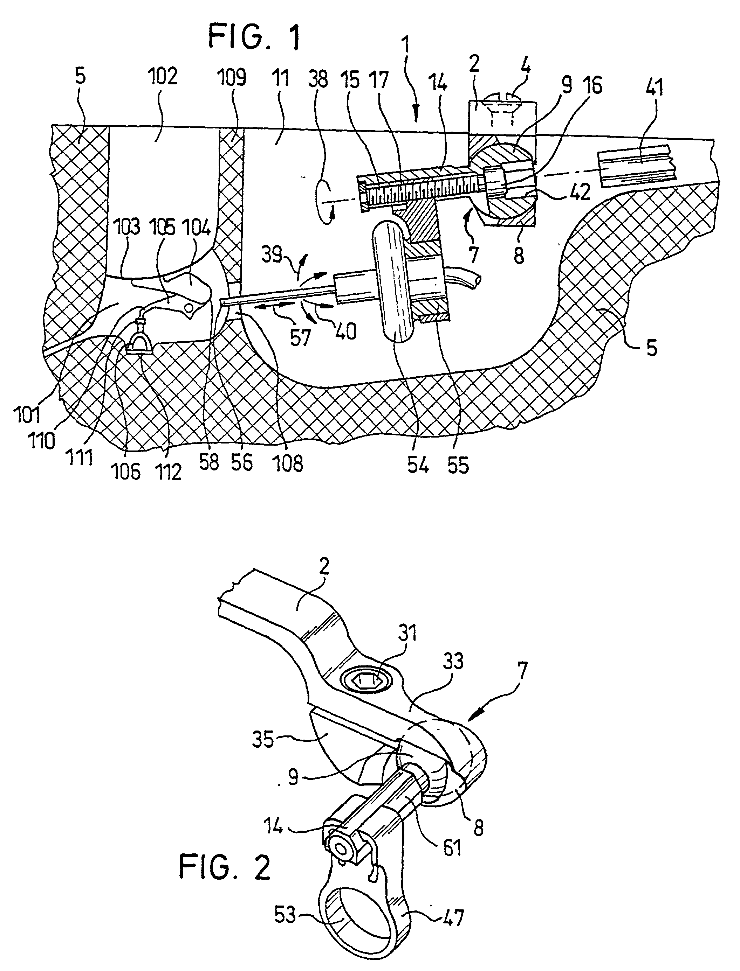

Das in den Figuren 1 bis 6 dargestellte implantierbare Positioniersystem 1 weist ein zur Knochenverankerung geeignetes Halteteil 2 mit Öffnungen 3 zum Durchstecken von Knochenschrauben 4 auf, mittels deren das Halteteil 2 auf einer Knochenoberfläche, wie z.B. dem Schädelknochen 5, festgeschraubt werden kann. Zu dem Positioniersystem 1 gehört ferner ein klemmbares Kugelgelenk 7, das eine an dem Halteteil 2 fest, vorzugsweise einstückig, angebrachte Kugelaufnahme 8 und eine von der Kugelaufnahme 8 zum Teil umgriffene Gelenkkugel 9 aufweist. Das Halteteil 2 ist in dem Bereich 10 des Übergangs zu der Kugelaufnahme 8 stufig abgesetzt. Durch dieses Absetzen wird nach dem Aufschrauben des Halteteils 2 auf die Knochenoberfläche und dem damit verbundenen Einbringen der Systemhauptkomponenten in eine zweckentsprechende Körperhöhle, beispielsweise eine Mastoidhöhle 11, ein Überstehen des Positioniersystems 1 über das Niveau der Knochenoberfläche vermieden.

Mit der Gelenkkugel 9 ist eine gerade Führungsschiene 14 fest, im gezeigten Ausführungsbeispiel einstückig, verbunden. In dem aus Gelenkkugel 9 und Führungsschiene 14 bestehenden Bauteil ist eine Gewindespindel 15 drehbar gelagert, die von der Führungsschiene 14 über einen Teil ihres Umfangs umfaßt wird. Die Gewindespindel 15 weist an ihrem kugelseitigen Ende einen Bedienkopf 16 mit einem Durchmesser auf, der größer als der Durchmesser des Gewinde tragenden Teils 17 der Gewindespindel 15 ist. Das von der Gelenkkugel 9 abgewendete Ende 18 der Gewindespindel 15 ist verjüngt. Mit diesem verjüngten Ende 18 ist eine Scheibe 19 fest verbunden, zum Beispiel durch Schweißen, Löten, Aufpressen oder dergleichen. Die Scheibe 19 ist in einer Ausnehmung 20 am freien Ende der Führungsschiene 14 versenkt angeordnet und mit Bezug auf die Führungsschiene 14 drehbar gelagert. Eine weitere Lagerfläche 21 ist an dem Ende des Gewindeteils 17 ausgebildet, das dem Bedienkopf 16 zugewendet ist. Diese Lagerfläche 21 wirkt mit einem Lagerbund 22 in der Gelenkkugel 9 zusammen. Die Gewindespindel 15 weist an der Treffstelle von Bedienkopf 16 und Gewindeteil 17 eine Schulter 23 auf, die am Boden einer den Bedienkopf 16 aufnehmenden Ausnehmung 24 der Gelenkkugel 9 anliegt. Die der Gelenkkugel 9 zugewendete Stirnfläche 25 der mit der Gewindespindel 15 fest verbundenen Scheibe 19 legt sich gegen den Boden der Ausnehmung 20 an. Die Schulter 23 und die Stirnfläche 25 sorgen im Zusammenwirken mit dem Boden der Ausnehmung 24 und dem Boden der Ausnehmung 20 für eine in Axialrichtung unverschiebbare Lagerung der Gewindespindel 15.

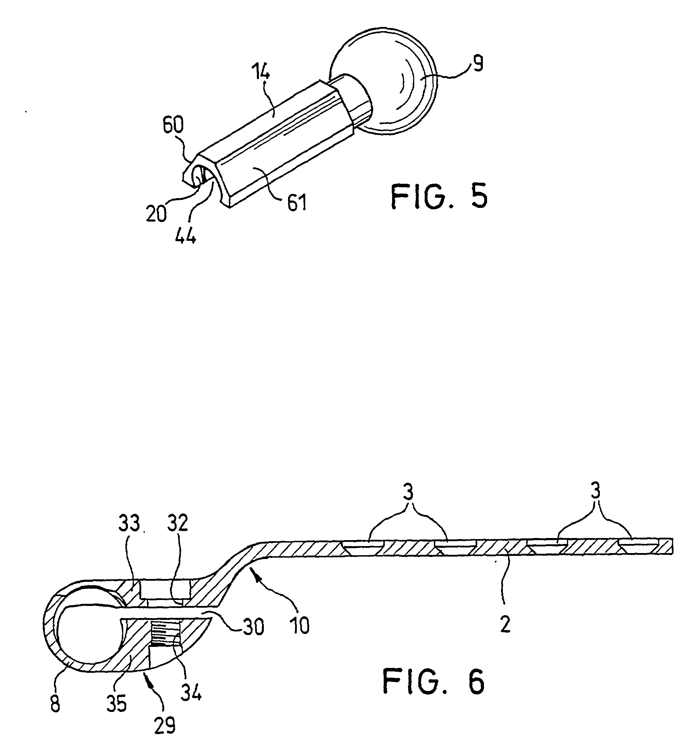

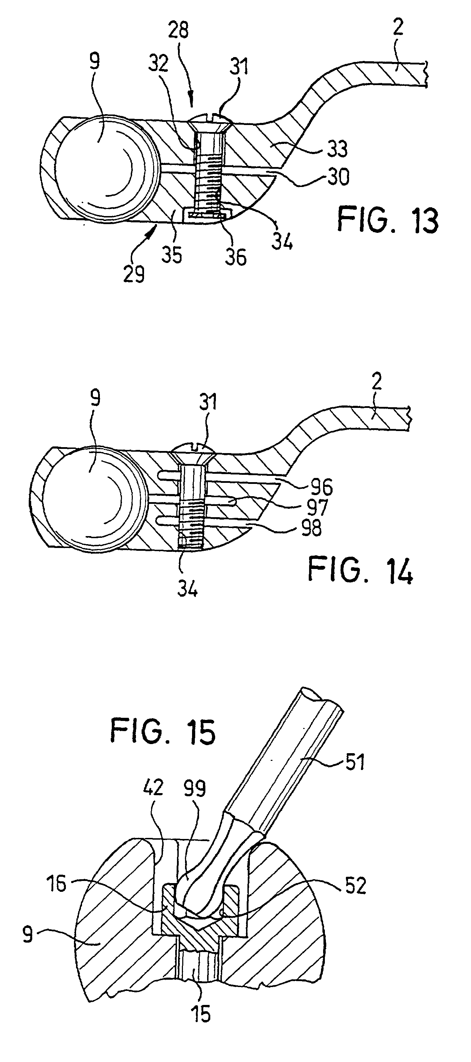

Zum Festklemmen des Kugelgelenks 7 in einer eingestellten Position ist ein insgesamt mit 28 bezeichneter Klemmechanismus vorgesehen. Bei dem Ausführungsbeispiel gemäß den Figuren 6 und 13 ist der Klemmechanismus als Schellen-Klemmvorrichtung aufgebaut, wobei die Kugelaufnahme 8 unter Bildung einer die Gelenkkugel 9 über einen Teil ihrer Umfangsfläche umschließenden Schelle 29 mit einem Schlitz 30 versehen ist. Zum Anpressen der Schelle 29 an die Gelenkkugel 9 läßt sich der Schlitz 30 verengen, indem eine Klemmschraube 31 angezogen wird, die durch eine Öffnung 32 einer ersten Schellenhälfte 33 gesteckt und in eine Gewindebohrung 34 einer zweiten Schellenhälfte 35 eingeschraubt ist. Durch Anziehen der Klemmschraube 31 wird die Gelenkkugel 9 fest in die Kugelsenkungen der Kugelaufnahme 8 gepreßt. Die Klemmschraube 31 und die Gewindebohrung 34 sind dabei vorzugsweise mit Feingewinde ausgestattet. Ein solches Gewinde erlaubt ein problemloses Aufbringen von hohen Klemmkräften; es ist ferner selbsthemmend, wodurch die Klemmschraube in der eingestellten Lage gesichert wird. Mittels des Klemmechanismus wird die momentane Position der Gelenkkugel 9, der an der Gelenkkugel befestigten Führungsschiene 14 und der Gewindespindel 15 auf einfache Weise räumlich fixiert. Durch Lösen der Klemmschraube 31 ist die Gelenkkugel 9 in ihrem Kugelsitz 8 in allen drei rotatorischen Freiheitsgraden 38, 39 und 40 frei schwenkbar. Dabei ist eine vollständige 360°-Drehung (Pfeil 38) um die Längsachse der Gewindespindel 15 möglich. Die Schwenkwinkel entlang der beiden weiteren rotatorischen Freiheitsgrade 39 und 40 betragen bei dem veranschaulichten Ausführungsbeispiel jeweils ungefähr 160°.

Zum Positionieren des Kugelgelenks 7 wird die Klemmschraube 31 vorzugsweise nur soweit gelöst, daß die Gelenkkugel 9 durch Reibkräfte in den Kugelsenkungen der Kugelaufnahme 8 noch gehalten wird. Eine unerwünschte Positionsänderung der momentanen Lage der Gewindespindel 15, zum Beispiel durch Verkipppen aufgrund der Gravitationskraft, wird somit vermieden. Das freie Ende der Klemmschraube 31 kann in der in Fig. 13 gezeigten Weise mit einem Verlierschutz 36 versehen sein, der das Klemmen und Freigeben des Kugelgelenks 7 erlaubt, ein Herausdrehen der Klemmschraube 31 aus der Gewindebohrung 34 jedoch verhindert. Der Verlierschutz 36 kann so ausgebildet und angebracht sein, daß er das Lösen der Klemmschraube 31 auf einen Wert begrenzt, bei dem die zuvor genannten Reibkräfte sichergestellt sind.

Das Verschwenken der Gelenkkugel 9 bei gelöstem Klemmechanismus 28 kann mittels eines Hilfswerkzeuges 41, beispielsweise in Form ein Inbusschlüssels, erfolgen. Dafür wird das Hilfswerkzeug 41 in eine komplementäre Aufnahmeöffnung 42 formschlüssig eingesteckt, die an der zum bedienenden Operateur zeigenden Seite der Gelenkkugel 9 in diese eingebracht ist.

Die im Querschnitt im wesentlichen schalenartig geformte Führungsschiene 14 begrenzt einen Freiraum 44 auf, der in axialer Richtung von der Scheibe 19 bis zu einer von der Gelenkkugel 9 gebildeten Anschlagfläche 45 verläuft. Mit dem Außengewinde des Spindelteils 17 steht ein Innengewinde einer Spindelmutter 46 in Eingriff, die Teil eines Schlittens 47 ist. Der Schlitten 47 erstreckt sich in radialer Richtung durch den Freiraum 44 hindurch.

Durch formschlüssiges Einsetzen eines Hilfswerkzeuges 51 (Fig. 15), beispielsweise eines Inbusschlüssels, in eine komplementäre Aufnahmeöffnung 52 des Bedienkopfes 16 der Gewindespindel 15 und durch manuelle Drehbewegung am Bedienkopf 16 wird entsprechend dem Gewindedrehsinn und der gewählten Gewindesteigung von Gewindespindel 15 und Spindelmutter 46 eine Axialverschiebung des Schlittens 47 entlang der Führungsschiene 14 bewirkt. Der Axialweg des Schlittens 47 ist am kugelgelenkseitigen Ende durch die Anschlagfläche 45 und am gegenüberliegenden Ende durch die Scheibe 19 begrenzt. Der Schlitten 5 läßt sich somit entlang der Führungsschiene 14 zwischen den Endanschlägen 19 und 45 stufenlos verfahren, und er hält aufgrund der Selbsthemmung der Gewindetriebs 15, 46 seine momentane Position bei. Der Verfahrweg des Schlittens 47 entlang der Führungsschiene 14 beträgt bei einer bevorzugten Ausführungsform 5 bis 10 mm. In den Figuren 1 bis 3 ist der Schlitten 47 in einer Zwischenstellung zwischen den beiden Endanschlägen 19 und 45 dargestellt.

Der Schlitten 47 besitzt eine Aufnahme 53, in die ein aktorisches oder sensorisches Mittel 54 spielfrei eingesetzt werden kann. Bei der veranschaulichten Ausführungsform hat letzteres eine Längsachse, die mindestens näherungsweise parallel zu der Längsachse der Gewindespindel 15 und der Führungsschiene 14 verläuft, gegenüber dieser aber in Querrichtung versetzt ist. Falls eine mechanische Entkoppelung oder eine elastische Lagerung zwischen Positioniersystem 1 und dem in der Aufnahme 53 fixierten Mittel 54 erforderlich ist, kann zwischen die Aufnahme 53 und das Mittel 54 ein elastisches bzw. federelastisches Zwischenstück 55 eingefügt sein.

Das freie Wirkende 56 des in die Aufnahme 53 des Schlittens 47 eingelegten Mittels 54 ist somit durch Drehen des Bedienkopfes 16 der Gewindespindel 15 parallel zu der Führungsschiene 14 in axialer Richtung 57 mit Bezug auf einen körperfesten Zielpunkt 58 im menschlichen Körper positionierbar.

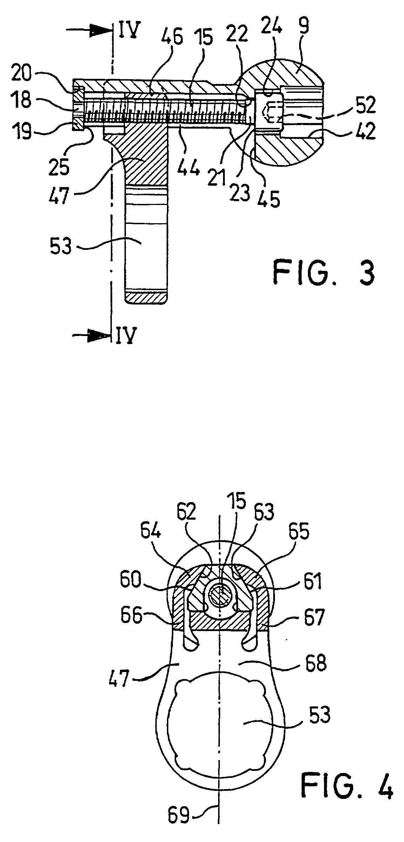

Zur exakten Führung des Schlittens 47 mit Bezug auf die Führungsschiene 14 sind an der Außenseite der Führungsschiene 14 zwei Führungs-Außenflächen 60 und 61 vorgesehen, die mit Führungs-Innenflächen 62 beziehungsweise 63 des Schlittens 47 in Gleiteingriff gehalten sind. Die Führungs-Innenflächen 62, 63 sind bei der veranschaulichten Ausführungsform von Wangen 64 beziehungsweise 65 gebildet, die an Federarmen 66 beziehungsweise 67 des Schlittens 47 angeformt und damit in einer zur Längsachse der Gewindespindel 15 senkrecht stehenden Ebene (der Zeichnungsebene der Fig. 4) federnd angeordnet sind. Die Federarme 66, 67 sind ihrerseits mit einem Schlittenkörper 68 einstückig verbunden, der auch die Spindelmutter 46 bildet.

Wie insbesondere aus Fig. 4 hervorgeht, liegen die Führungs-Innenflächen 62, 63 des Schlittens 47 und die Führungs-Außenflächen 60, 61 der Führungsschiene 14 jeweils einander diametral gegenüber, und sie sind spiegelsymmetrisch zu einer die Längsachse der Spindelmutter 46 enthaltenden Längssymmetrieebene 69 des Schlittens beziehungsweise spiegelsymmetrisch zu einer mit der Ebene 69 zusammenfallenden, die Längsachse der Gewindespindel 15 enthaltenden Längssymmetrieebene der aus Führungsschiene 14 und Gewindespindel 15 bestehenden Baugruppe angeordnet. Dabei Schließen die Führungs-Innenflächen 62, 63 des Schlittens 47 und die Führungs-Außenflächen 60, 61 der Führungsschiene 14 mit der Längssymmetrieebene 69 des Schlittens beziehungsweise der Längssymmetrieebene der aus Führungsschiene und Gewindespindel 15 bestehenden Baugruppe vorzugsweise jeweils einen Winkel im Bereich von 10° bis 60° ein. Diese Schrägstellung der Führungsflächen sorgt für eine selbsttätige Zentrierung des Schlittens 47 auf der Führungsschiene 14, und sie bewirkt, daß die Gewindespindel 15 keine Führungsfunktion hat, sondern nur dem Vortrieb des Schlittens 47 dient.

Im spannungsfreien Zustand der Federarme 66, 67, das heißt bei von der Gewindespindel 15 abgenommenem Schlitten 47, haben die Führungs-Innenflächen 62, 63 vorzugsweise einen gegenseitigen Abstand, der um ein vorgegebenes Maß kleiner ist als der gegenseitige Abstand der Führungs-Außenflächen 60, 61. Dadurch werden die Führungs-Innenflächen 62, 63 unter Ausgleich von Fertigungstoleranzen zwangsweise mit vorgewählter Vorspannkraft an die Führungs-Außenflächen 60, 61 angedrückt.

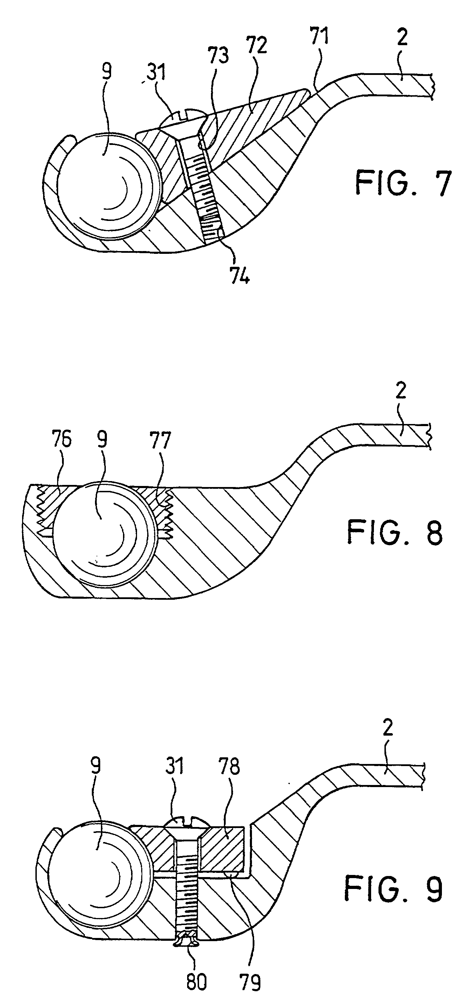

In den Figuren 7 bis 12 und 14 sind verschiedene abgewandelte Ausführungsformen des Klemmechanismus 28 veranschaulicht.

Fig. 7 zeigt eine Keil-Klemmvorrichtung, die eine auf einer schrägen Ebene 71 gleitende Druckkalotte 72 aufweist. Die Klemmschraube 31 ist durch eine Bohrung 73 der Druckkalotte 72 gesteckt und in eine mit der Bohrung 73 axial ausgerichtete Gewindebohrung 74 des Halteteils 2 eingeschraubt. Durch Anziehen der Klemmschraube 31 kann die Druckkalotte 72 gegen die Gelenkkugel 9 angepreßt werden.

Bei der Ausführungsform gemäß Fig. 8 ist der Klemmechanismus als Ring-Klemmvorrichtung mit einem Klemmring 76 ausgebildet. Der Klemmring 76 ist in eine Gewindebohrung 77 der Kugelaufnahme eingeschraubt, und er wird zum Fixieren des Kugelgelenks in der eingestellten Position gegen die Gelenkkugel 9 angepreßt.

Der in Fig. 9 dargestellte Klemmechanismus bildet einen Hebel-Klemmvorrichtung mit einer als Hebel wirkenden Druckkalotte 78. Die Druckkalotte 78 weist an ihrer der dem Halteteil 2 zugewendeten Seite einen als Hebel-Gegenlager dienenden Vorsprung 79 auf. Durch Anziehen der Klemmschraube 31 wird die Druckkalotte 78 um das Hebel-Gegenlager geschwenkt und dabei gegen die Gelenkkugel 9 angepreßt. Die Klemmschraube 31 ist an ihrem von der Druckkalotte 78 abliegenden und aus dem Halteteil 2 vorstehenden Ende 80 aufgeweitet, um für einen Verlierschutz der Klemmschraube 31 zu sorgen.

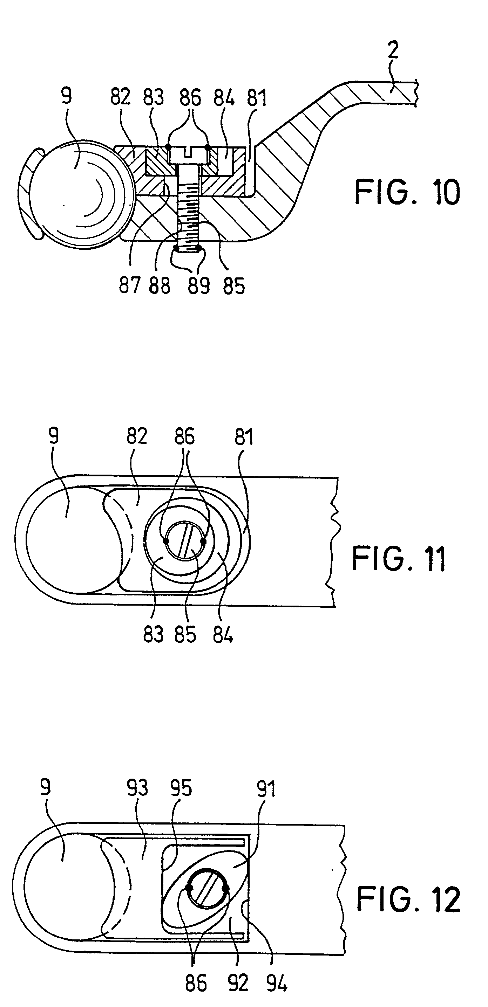

Die Figuren 10 und 11 zeigen eine Exzenter-Klemmvorrichtung mit einer Druckkalotte 82, die in einer Ausnehmung 81 der Kugelaufnahme mit Bezug auf die Gelenkkugel 9 verschiebbar angeordnet ist. Dabei wirkt die Druckkalotte 82 mit einer Exzenterscheibe 83 zusammen, die in eine Ausnehmung 84 der Druckkalotte 82 eingreift und die mit einer Klemmschraube 85, beispielsweise mit Hilfe von Punktscheißungen 86, drehfest verbunden ist. Die Klemmschraube 85 ist durch eine Öffnung 87 der Druckkalotte 82 hindurchgesteckt, die einen ausreichend großen Durchmesser hat, um eine Verstellbewegung der Druckkalotte 82 gegenüber der Gelenkkugel 9 zu gestatten. Bei Drehen der in eine Gewindebohrung 88 der Kugelaufnahme eingeschraubten Klemmschraube 85 wird die Exzenterscheibe 83 um eine Drehachse verschwenkt, die von der Klemmschraube 85 bestimmt ist. Durch Anlage der Außen-Umfangsfläche der Exzenterscheibe 83 an der Innen-Umfangsfläche der Ausnehmung 84 der Druckkalotte 82 läßt sich die Druckkalotte 82 in der Ausnehmung 81 verschieben und gegen die Gelenkkugel 9 anpressen. Die Klemmschraube 85 steht aus der Gewindebohrung 88 vor. Das vorstehende Ende der Klemmschraube 85 ist mit mindestens einem Schweißpunkt 89 versehen, der als Verlierschutz für die Klemmschraube 85 dient.

In Fig. 12 ist eine abgewandelte Ausführungsform einer Exzenter-Klemmvorrichtung dargestellt. Auch in diesem Fall ist die Klemmschraube 85 mit einer Exzenterscheibe 91 drehfest verbunden, beispielsweise mit Hilfe der Punktscheißungen 86. Die Exzenterscheibe 91 sitzt in einer Ausnehmung 92 der Kugelaufnahme, in der auch eine Druckkalotte 93 mit Bezug auf die Gelenkkugel 9 verschiebbar angeordnet ist. Bei Drehen der in eine Gewindebohrung der Kugelaufnahme eingeschraubten Klemmschraube 85 wird die Exzenterscheibe 91 um die von der Klemmschraube 85 bestimmte Drehachse verschwenkt. Durch Abstützen der Exzenterscheibe 91 einerseits an einer Anlagefläche 94 der Kugelaufnahme und andererseits an einer Anlagefläche 95 der Druckkalotte 93 kann die Druckkalotte 93 gegen die Gelenkkugel 9 angedrückt werden.

Fig. 14 zeigt eine Schellen-Klemmvorrichtung ähnlich der bereits anhand der Fig. 13 erläuterten Klemmvorrichtung, jedoch mit dem Unterschied, daß die die Gelenkkugel 9 federnd umschließende Kugelaufnahme mehrfach geschlitzt ist (Schlitze 96, 97, 98). Eine solche Ausbildung hat gegenüber der Anordnung nach Fig. 13 den Vorteil, daß die beim Anziehen der Klemmschraube 31 auf die Gelenkkugel 9 ausgeübte Querkraft, welche die Gelenkkugel 9 gegenüber dem Halteteil 2 seitlich zu verschieben sucht, noch kleiner gehalten ist.

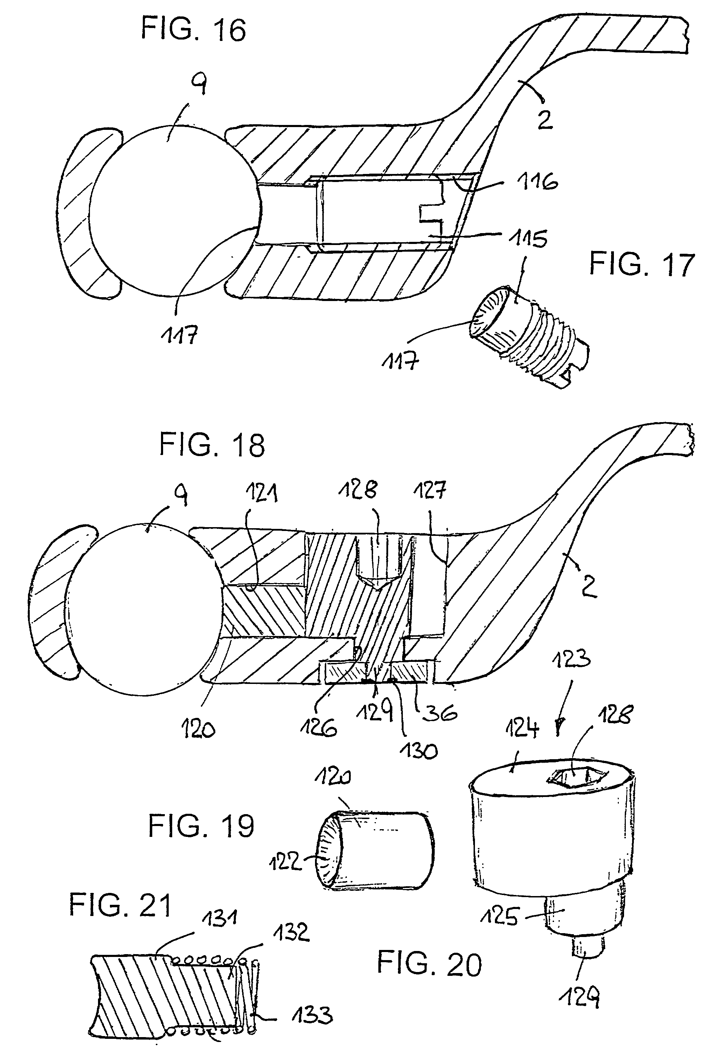

In den Figuren 16 und 17 ist eine Druckstift- Klemmvorrichtung gezeigt, die einen Außengewinde tragenden Druckstift 115 aufweist, dessen Längsachse senkrecht zu der Außenfläche der Gelenkkugel 9 steht. Der Druckstift 115 ist in eine Gewindebohrung 116 des Halteteils 2 eingeschraubt. Sein der Gelenkkugel 9 zugewendetes Ende 117 ist kalottenförmig mit einem Krümmungsradius vertieft, welcher dem Außendurchmesser der Gelenkkugel 9 entspricht. Beim Eindrehen des Druckstiftes 115 in die Gewindebohrung 116 wird der Druckstift 115 gegen die Gelenkkugel 9 angepreßt, wodurch diese in der eingestellten Lage festgeklemmt wird.

Die Figuren 18 bis 20 zeigen eine weitere Druckstift- Klemmvorrichtung. Bei dieser ist ein Druckstift 120 in einer zu der Außenfläche der Gelenkkugel 9 senkrecht stehenden Bohrung 121 des Halteteils 2 verschiebbar angeordnet. Der Druckstift 120 weist ein der Gelenkkugel 9 zugewendetes Ende 122 auf, das wie das Ende 117 Des Stiftes 115 kalottenförmig mit einem Krümmungsradius vertieft ist, der dem Außendurchmesser der Gelenkkugel 9 entspricht. Der Druckstift 120 wird zum Klemmen der Gelenkkugel 9 gegen diese mittels eines Exzenters 123 angedrückt, der dazu mit dem von der Gelenkkugel 9 abgewendeten Ende des Druckstiftes 120 zusammenwirkt. Der in Fig. 20 perspektivisch dargestellte Exzenter 123 weist einen zylindrischen Exzenterkörper 124 auf, dessen Längsachse gegenüber der Längsachse eines verjüngten Lagerrabschnittes 125 des Exzenters 123 seitlich versetzt ist und an dessen Umfangsfläche sich der Druckstift 120 abstützt. Der Exzenter 123 ist über seinen Lagerrabschnitt 125 in einer zu der Bohrung 121 senkrecht verlaufenden Bohrung 126 des Halteteils 2 drehbar gelagert. Der Exzenterkörper 124 sitzt in einer Bohrung 127 des Halteteils 2, die koaxial zu der Bohrung 126 angeordnet ist und deren Durchmesser ausreichend groß ist, um ein Verschwenken des Exzenters 123 zu erlauben. Ein solches Verschwenken geschieht mittels eines (nicht dargestellten) Werkzeugs, das in eine Mehrkantbohrung 128 des Exzenterkörpers 124 eingesetzt wird. Mit einem verjüngten Endabschnitt 129 an der von dem Exzenterkörper 124 abgewendeten Seite des Lagerrabschnittes 125 ist eine Verlierschutzscheibe 36 fest verbunden, zum Beispiel mit Hilfe von Laserschweißungen, die bei 130 angedeutet sind. Die Verlierschutzscheibe 36 hat einen Durchmesser, der größer als der Durchmesser der Bohrung 126 ist.

Anstelle des Druckstiftes 120 der Figuren 18 und 19 kann gemäß Fig. 21 auch ein Druckstift 131 vorgesehen sein, der einen von der Gelenkkugel 9 abgewendeten verjüngten Abschnitt 132 aufweist, auf dem eine Schrauben-Druckfeder 133 sitzt. Diese Feder spannt den Druckstift 131 in Richtung auf die Gelenkkugel 9 vor und sorgt auf diese Weise für eine Vorfixierung der Gelenkkugel.

Das dem Drehen der Gewindespindel 15 dienende Hilfswerkzeug 51 hat, wie in Fig. 15 gezeigt, vorteilhaft einen kugeligen Kopf 99 mit Mehrkantprofil, beispielsweise Sechskantprofil. Der Werkzeugkopf 99 kann mit der ein komplementäres Mehrkantprofil aufweisenden Aufnahmeöffnung 52 im Bedienkopf 16 der Gewindespindel 15 in Formschluß-Eingriff gebracht werden. Weil der Kopf 99 kugelig ist und die Öffnungsweite der Aufnahmeöffnung 42 der Gelenkkugel größer ist als die Öffnungsweite der Aufnahmeöffnung 52, läßt sich die Gewindespindel 15 auch dann sicher Drehen, wenn es die Platzverhältnisse am Implantationsort nicht erlauben, die Achse des Hilfswerkzeugs 51 mit der Achse der Gewindespindel 15 auszurichten.

Fig. 1 läßt mögliche Ankoppelpunkte im Mittelohr 101 erkennen, das vom äußeren Gehörgang 102 durch das Trommelfell 103 abgegrenzt ist. Als Aktor 54 wird bei der Anordnung nach Fig. 1 ein zur vibratorischen Stimulierung der Ossikelkette geeigneter Hörgerätewandler in der Aufnahme 53 des Schlittens 47 gehalten. Der Hörgerätewandler ist Bestandteil eines teilweise oder vollständig implantierbaren Hörgerätes. Bei dieser bevorzugten Ausführung ist der körperfeste Zielpunkt 58 ein Punkt auf der Hammer 104, Amboß 105 und Steigbügel 106 umfassenden Ossikelkette.

Die Körperöffnung, in die das Positioniersystem 1 gemäß dieser bevorzugten Ausführungsform implantiert und intraoperativ mit Hilfswerkzeugen positioniert und fixiert werden kann, ist die Mastoidhöhle 11 im Schädelknochen 5. Das Halteteil 2 ist auf die Oberfläche des an die Mastoidhöhle angrenzenden Schädelknochens 5 aufgeschraubt. Das freie Wirkende 56 des aktorischen Hörgerätewandlers 54 reicht durch einen Knochendurchbruch 108 der hinteren Gehörgangswand 109 in das Mittelohr 101. Je nach anatomisch vorgefundener Situation von Mastoidhöhle 11, hinterer Gehörgangswand 109 und Mittelohr 101 entscheidet der Operateur über den jeweils am besten geeigneten Zielpunkt 58 auf der Ossikelkette.

Zur Ankopplung an den Amboßkörper 105 kann als Knochendurchbruch 108 für das freie Wirkende 56 des Hörgerätewandlers 54 ein natürlich vorhandener Kanal in der hinteren Gehörgangswand 109, der Aditus ad antrum, genutzt werden. Für die Ankopplung an den langen Amboßfortsatz 110, den Processus lenticularis 111 sowie an Strukturen des Steigbügels 106, z.B. die Steigbügelplatte 112, muß ein geeigneter Knochendurchbruch in der hinteren Gehörgangswand 109 gebohrt werden. Dieser Durchbruch wird im Chorda-Fazialis-Winkel geschaffen und besitzt zweckmäßig einen Durchmesser von etwa 2 mm.

Die Ankopplung des freien Wirkendes 56 des Hörgerätewandlers 54 an die Gehörknöchelchen des Mittelohres (Hammer, Amboß, Steigbügel) sowie an Strukturen des Innenohres und des Vestibularorgans kann in beliebiger bekannter Weise erfolgen.

Geeignete aktive oder passive, aktorische bzw. sensorische Mittel 54 für die Anwendung des Positioniersystems 1 sind unter anderem aktive elektromechanische Hörgerätewandler zur elektromechanischen Stimulation der Gehörknöchelchenkette, Erregerspulen zur elektromagnetischen Stimulation von ossikelkettenfest fixierten Permanentmagneten, Lichtleiter zur Führung von chirurgischem Laserlicht (z.B. zum Schneiden, Bohren, Koagulieren oder Veröden von Gewebe oder Knochenstrukturen), Lichtleiter zur Führung von meßtechnischem Laserlicht (Laser-Doppler-Vibrometrie), flexible Miniaturendoskope zur Inspektion beliebiger Schädelregionen, Sondenmikrophone und kleine Schallquellen zur intraoperativen Audiometrie (Hörschwellenbestimmung, Ableitung von otoakustischen Emissionen) sowie Elektroden zur Ableitung von elektrocochleographischen Körperpotentialen (z.B. Summenaktionspotential oder Mikrophonpotential) bzw. zur Elektrostimulation im Rahmen präoperativer Hörtests vor Implantation von Cochlear Implants (Promontorialtest).

## Patentansprüche

1. Dauerhaft implantierbares fixierbares Positioniersystem (1) für die feste, spielfreie Anbindung an einem Knochen des menschlichen Körpers, insbesondere dem Schädelknochen, mit :
- einem eine Gelenkkugel (9) und eine Kugelaufnahme (8) aufweisenden Kugelgelenk (7), dessen Gelenkkugel mit Bezug auf die Kugelaufnahme mittels eines Hilfswerkzeugs (41) manuell schwenkbar und durch einen Klemmechanismus (28) fixierbar ist,
- einer mit der Gelenkkugel (9) des Kugelgelenks (7) fest verbundenen geraden Führungsschiene (14),
- einer mit Bezug auf die Führungsschiene (14) drehbar, aber in Axialrichtung unverschiebbar gelagerten, Außengewinde aufweisenden Gewindespindel (15),
- einem Schlitten (47), der eine Spindelmutter (46) mit einem mit dem Außengewinde der Gewindespindel (15) in Gewindeeingriff stehenden Innengewinde aufweist und der durch manuelles Drehen der Gewindespindel (15) mit einem Hilfswerkzeug (51) zwischen Endanschlägen (19, 45) entlang der Führungsschiene (14) frei positionierbar ist,
- einer an dem Schlitten (47) angebrachten Aufnahme (53) für ein zu positionierendes bzw. fixierendes aktorisches oder sensorisches Mittel (54), und
- einem an dem Knochen anschraubbaren Halteteil (2), an dem die Kugelaufnahme (8) und der Klemmechanismus (28) des Kugelgelenks (7) angebracht sind,
**dadurch gekennzeichnet, daß**
die Führungsschiene (14) Führungs-Außenflächen (60, 61) aufweist, die in Gleiteingriff mit Führungs-Innenflächen (62, 63) des Schlittens (47) gehalten sind, und die Führungs-Innenflächen (62, 63) von Wangen (64, 65) des Schlittens (47) gebildet sind, die mit Bezug auf die Spindelmutter (46) in einer zur Längsachse der Gewindespindel (15) senkrecht stehenden Ebene federnd angeordnet sind.

2. Positioniersystem nach Anspruch 1, **dadurch gekennzeichnet, daß** im spannungsfreien Zustand die Führungs-Innenflächen (62, 63) einen gegenseitigen Abstand haben, der um ein vorgegebenes Maß kleiner ist als der gegenseitige Abstand der Führungs-Außenflächen (60, 61).

3. Positioniersystem nach Anspruch 1 oder 2, **dadurch gekennzeichnet, daß** die Wangen (64, 65) an Federarmen (66, 67) angeformt sind, die mit einem die Spindelmutter (46) aufweisenden Schlittenkörper (68) einstückig verbunden sind.

4. Positioniersystem nach einem der vorhergehenden Ansprüche, **dadurch gekennzeichnet, daß** die Führungs-Innenflächen (62, 63) des Schlittens (47) und die Führungs-Außenflächen (60, 61) der Führungsschiene (14) jeweils einander diametral gegenüberliegen und spiegelsymmetrisch zu einer die Längsachse der Spindelmutter (46) enthaltenden Längssymmetrieebene (69) des Schlittens beziehungsweise spiegelsymmetrisch zu einer die Längsachse der Gewindespindel (15) enthaltenden Längssymmetrieebene der aus Führungsschiene und Gewindespindel bestehenden Baugruppe angeordnet sind.

5. Positioniersystem nach Anspruch 4, **dadurch gekennzeichnet, daß** die Führungs-Innenflächen (62, 63) des Schlittens (47) und die Führungs-Außenflächen (60, 61) der Führungsschiene (14) mit der Längssymmetrieebene (69) des Schlittens beziehungsweise der Längssymmetrieebene der aus Führungsschiene und Gewindespindel (15) bestehenden Baugruppe jeweils einen Winkel im Bereich von 10° bis 60° einschließen.

6. Positioniersystem nach einem der vorhergehenden Ansprüche, **dadurch gekennzeichnet, daß** die Spindelmutter (46) und die Gewindespindel (15) mit selbsthemmendem Gewinde versehen sind.

7. Positioniersystem nach einem der vorhergehenden Ansprüche, **dadurch gekennzeichnet, daß** die Gewindespindel (15) an ihrem kugelseitigen Ende und an ihrem von der Gelenkkugel (9) abliegenden Ende in der die Gelenkkugel und die Führungsschiene (14) umfassenden Anordnung drehbar gelagert ist.

8. Positioniersystem nach einem der vorhergehenden Ansprüche, **dadurch gekennzeichnet, daß** die Gelenkkugel (9) eine Aufnahme (42) zum Ansetzen des dem Schwenken der Gelenkkugel dienenden Hilfswerkzeugs (41) aufweist.

9. Positioniersystem nach einem der vorhergehenden Ansprüche, **dadurch gekennzeichnet, daß** die Gewindespindel (15) an ihrem der Gelenkkugel (9) zugewendeten Ende eine Aufnahme (52) zum Ansetzen des dem Drehen der Gewindespindel dienenden Hilfswerkzeugs (51) aufweist.

10. Positioniersystem nach Ansprüchen 8 und 9, **dadurch gekennzeichnet, daß** die Aufnahmen (42, 52) zueinander koaxial angeordnet sind und die Aufnahme (52) zum Ansetzen des dem Drehen der Gewindespindel (15) dienenden Hilfswerkzeugs (51) durch die Aufnahme (42) zum Ansetzen des dem Schwenken der Gelenkkugel (9) dienenden Hilfswerkzeugs (41) hindurch zugänglich ist.

11. Positioniersystem nach einem der vorhergehenden Ansprüche, **dadurch gekennzeichnet, daß** zum Betätigen des Klemmechanismus ein einziges Stellelement (31, 76, 85, 115, 123) vorgesehen ist.

12. Positioniersystem nach einem der vorhergehenden Ansprüche, **dadurch gekennzeichnet, daß** der Klemmechanismus als Keil-Klemmvorrichtung ausgebildet ist.

13. Positioniersystem nach Ansprüchen 11 und 12, **dadurch gekennzeichnet, daß** die Keil-Klemmvorrichtung eine auf einer schrägen Ebene (71) gleitende Druckkalotte (72) aufweist, die durch Anziehen einer das Stellelement bildenden Klemmschraube (31) gegen die Gelenkkugel (9) anpreßbar ist.

14. Positioniersystem nach einem der Ansprüche 1 bis 11, **dadurch gekennzeichnet, daß** der Klemmechanismus als Ring-Klemmvorrichtung ausgebildet ist.

15. Positioniersystem nach Ansprüchen 11 und 14, **dadurch gekennzeichnet, daß** die Ring-Klemmvorrichtung einen in ein Gewinde (77) der Kugelaufnahme einschraubbaren und gegen die Gelenkkugel (9) anpreßbaren Klemmring (76) aufweist, der das Stellelement bildet.

16. Positioniersystem nach einem der Ansprüche 1 bis 11, **dadurch gekennzeichnet, daß** der Klemmechanismus als Hebel-Klemmvorrichtung ausgebildet ist.

17. Positioniersystem nach Ansprüchen 11 und 16, **dadurch gekennzeichnet, daß** die Hebel-Klemmvorrichtung eine als Hebel wirkende Druckkalotte (78) aufweist, die durch Anziehen einer das Stellelement bildenden Klemmschraube (31) um ein Hebel-Gegenlager (79) schwenkbar und gegen die Gelenkkugel (9) anpreßbar ist.

18. Positioniersystem nach einem der Ansprüche 1 bis 11, **dadurch gekennzeichnet, daß** der Klemmechanismus als Exzenter-Klemmvorrichtung ausgebildet ist.

19. Positioniersystem nach Ansprüchen 11 und 18, **dadurch gekennzeichnet, daß** die Exzenter-Klemmvorrichtung eine Druckkalotte (82, 93) aufweist, die mit einer Exzenterscheibe (83, 91) zusammenwirkt, die ihrerseits mit einer das Stellelement bildenden Klemmschraube (85) drehfest verbundenen ist, und daß die Druckkalotte (82, 93) durch Drehen der Klemmschraube (85) und damit der Exzenterscheibe (83, 91) gegen die Gelenkkugel (9) anpreßbar ist.

20. Positioniersystem nach einem der Ansprüche 1 bis 11, **dadurch gekennzeichnet, daß** der Klemmechanismus als Schellen-Klemmvorrichtung ausgebildet ist.

21. Positioniersystem nach Ansprüchen 11 und 20, **dadurch gekennzeichnet, daß** die Kugelaufnahme (8) derart geschlitzt ist, daß sie die Gelenkkugel (9) federnd umschließt, und die Kugelaufnahme durch Anziehen einer das Stellelement bildenden Klemmschraube (31) gegen die Gelenkkugel anpreßbar ist.

22. Positioniersystem nach einem der Ansprüche 1 bis 11, **dadurch gekennzeichnet, daß** der Klemmechanismus als Druckstift- Klemmvorrichtung ausgebildet ist.

23. Positioniersystem nach Ansprüchen 11 und 22, **dadurch gekennzeichnet, daß** ein das Stellelement bildender Gewinde-Druckstift (115) in eine Gewindebohrung (116) des Halteteils (2) eingeschraubt und durch Drehen gegen die Gelenkkugel (9) anpreßbar ist.

24. Positioniersystem nach Ansprüchen 11 und 22, **dadurch gekennzeichnet, daß** ein das Stellelement bildender Druckstift (120, 131) durch Verschwenken eines Exzenters (123) gegen die Gelenkkugel (9) anpreßbar ist.

25. Positioniersystem nach Anspruch 24, **dadurch gekennzeichnet, daß** der Druckstift (131) in Richtung auf die Gelenkkugel (9) federnd vorgespannt ist.

26. Positioniersystem nach einem der vorhergehenden Ansprüche, **dadurch gekennzeichnet, daß** das Stellelement (31, 85) mit einem Verlierschutz (36, 80, 89) versehen ist.

27. Positioniersystem nach einem der vorhergehenden Ansprüche, **dadurch gekennzeichnet, daß** das dem Drehen der Gewindespindel (15) dienende Hilfswerkzeug (51) einen kugeligen Kopf (99) mit Mehrkantprofil hat, der mit einer ein komplementäres Mehrkantprofil aufweisenden Ausnehmung (52) der Gewindespindel (15) in Formschluß-Eingriff bringbar ist.

28. Positioniersystem nach einem der vorhergehenden Ansprüche, **dadurch gekennzeichnet, daß** es aus implantierbaren Metallen, wie z.B. Reintitan, seinen implantierfähigen Legierungen sowie Implantat-Edelstählen, gefertigt ist.

29. Positioniersystem nach einem der vorhergehenden Ansprüche, **dadurch gekennzeichnet, daß** die aktorischen oder sensorischen Mittel (54) zur Diagnose, Therapie und/oder für chirurgische Applikationen für die temporäre oder dauerhafte Implantation der genannten Mittel ausgelegt sind.

30. Positioniersystem nach einem der vorhergehenden Ansprüche, **dadurch gekennzeichnet, daß** es sich bei dem aktorischen Mittel (54) um einen implantierbaren elektromechanischen Hörgerätewandler handelt.

31. Positioniersystem nach einem der vorhergehenden Ansprüche, **dadurch gekennzeichnet, daß** es zum Positionieren und Ankoppeln eines daran befestigten Hörgerätewandlers (54) zu jedem beliebigen Punkt des Mittelohres einschließlich jeder beliebigen Ankoppelstelle an das Innenohr, wie einem natürlichen oder artifiziellen Fenster, als körperfestem Zielpunkt (58) und zum Fixieren des Hörgerätewandlers in dieser Lage ausgelegt ist.

32. Positioniersystem nach Anspruch 31, **dadurch gekennzeichnet, daß** es zum Positionieren und Ankoppeln eines daran befestigten Hörgerätewandlers (54) zu Hammer (104), Amboß (105) oder Steigbügel (106) als körperfestem Zielpunkt (58) und zum Fixieren des Hörgerätewandlers in dieser Lage ausgelegt ist.

33. Positioniersystem nach Anspruch 31 oder 32, **dadurch gekennzeichnet, daß** der Hörgerätewandler (54) als aktorische Komponente eines teilweise oder vollständig implantierbaren Hörgerätes vorgesehen ist.

34. Positioniersystem nach einem der vorhergehenden Ansprüche, **dadurch gekennzeichnet, daß** das Positioniersystem (1) aufgrund der Freiheitsgrade des Kugelgelenks (7) und der aus Spindelmutter (46) und Gewindespindel (15) bestehenden Anordnung zusammengefaßt für eine axiale (57) und drei rotatorische (38, 39, 40) Bewegungen des aktorischen oder sensorischen Mittels (54) und seines freien Wirkendes (56) in Körperöffnungen des menschlichen Körpers ausgelegt ist.

35. Positioniersystem nach einem der vorhergehenden Ansprüche, **dadurch gekennzeichnet, daß** es bei gelöster Klemmung des Kugelgelenks (7) für eine Verstellung aller drei rotatorischen Freiheitsgrade (38, 39, 40) des Kugelgelenks mittels des mit der Gelenkkugel (9) in Eingriff bringbaren Hilfswerkzeugs (41) ausgelegt ist.

36. Positioniersystem nach einem der Ansprüche 30 und 31, **dadurch gekennzeichnet, daß** die momentane Position der drei rotatorischen Freiheitsgrade (38, 39, 40) bei gelöstem Klemmechanismus (28) des Kugelgelenks (7) durch Reibkräfte gesichert ist und nach dem Schließen des Klemmechanismus dauerhaft beibehalten wird.

37. Positioniersystem nach einem der vorhergehenden Ansprüche, **dadurch gekennzeichnet, daß** die Bedienteile (31, 41, 51, 77, 85) zur manuellen Positionierung des Kugelgelenks (7) und des Schlittens (47) sowie zur Klemmung des Kugelgelenks vom Körper des Patienten weg zum Operateur zeigen.

38. Positioniersystem nach einem der vorhergehenden Ansprüche, **dadurch gekennzeichnet, daß** die Konstruktion und die geometrischen Abmessungen des Positioniersystems (1) derart gestaltet sind, daß der bedienende Operateur sowohl bei Arbeiten mit bloßem Auge als auch bei Verwendung eines Mikroskopes freie Sicht auf mindestens das freie Wirkende (56) des aktorischen oder sensorischen Mittels (54) sowie den Implantationsbereich samt Zielpunkt (58) im Körper des Patienten hat.

## Claims

1. Permanently implantable positioning system (1) for a rigid and free of play connection to a bone of the human body, especially the cranial bone, comprising:
a ball-and-socket joint (7) comprising a ball (9) and a socket (8), the ball being manually pivotable relative to the socket by means of an accessory tool (41) and being fixable by means of a clamp mechanism (28),
a straight guide rail (14) which is fixed to the ball (9) of the ball-and-socket joint (7),
a threaded spindle (15) having an outer thread and being supported rotatably relative to the guide rail (14), but unmovably in axial direction,
a carriage (47) comprising a spindle nut (46) having an inner thread that is engaged by the outer thread of the threaded spindle (15) in a manner that the carriage is freely positionable between two limit stops (19, 45) along the guide rail (14) by turning the threaded spindle (15) manually by means of an accessory tool (51),
a receptacle (53) attached to the carriage (47) for receiving an actuator or sensor means (54) which is to be positioned and fixed respectively, and
a holding device (2) which is adapted to be screwed down to the bone and at which the socket (8) and the clamp mechanism (28) of the ball-and-socket joint (7) are fixed,
**characterized in that**
the guide rail (14) includes outer guide surfaces (60, 61) which are held in sliding engagement with inner guide surfaces (62, 63) of the carriage (47) and the inner guide surfaces (62, 63) are formed by cheeks (64, 65) of the carriage (47), the cheeks being elastically movable relative to the spindle nut (46) in a plane perpendicular to the longitudinal axis of the threaded spindle (15).

2. Positioning system according to claim 1, **characterized in that** the inner guide surfaces (62, 63) span in an untensioned state a mutual distance which is by a predetermined value smaller than the mutual distance between the outer guide surfaces (60,61).

3. Positioning system according to claim 1 or 2, **characterized in that** the cheeks (64, 65) are molded onto spring arms (66, 67) which are integrally connected to a carriage body (68) comprising the spindle nut (46).

4. Positioning system according to anyone of the preceding claims, **characterized in that** the inner guide surfaces (62, 63) of the carriage (47) and the outer guide surfaces (60, 61) of the guide rail (14) are arranged diametrically opposed to each other and in mirror symmetry with respect to a longitudinal symmetry plane (69) of the carriage, which plane includes the longitudinal axis of the spindle nut (46), and in mirror symmetry with respect to a longitudinal symmetry plane of the subassembly consisting of guide rail and spindle nut, which plane includes the longitudinal axis of the threaded spindle (15), respectively.

5. Positioning system according to claim 4, **characterized in that** the inner guide surfaces (62, 63) of the carriage (47) and the outer guide surfaces (60, 61) of the guide rail (14) are angled in a range between 10° and 60° with respect to the longitudinal symmetry plane (69) of the carriage and the longitudinal symmetry plane of the subassembly consisting of guide rail and the threaded spindle (15), respectively.

6. Positioning system according to one of the preceding claims, **characterized in that** the spindle nut (46) and the threaded spindle (15) include self-locking threads.

7. Positioning system according to one of the preceding claims, **characterized in that** the threaded spindle (15) is rotatably supported in its ball-sided end and in its end facing away from the ball (9) in the arrangement comprising ball and guide rail (14).

8. Positioning system according to one of the preceding claims, **characterized in that** the ball (9) includes a receptacle (42) for receiving the accessory tool (41) for pivoting the ball.

9. Positioning system according to anyone of the preceding claims, **characterized in that** the threaded spindle (15) includes a receptacle (52) at its ball-sided end for receiving the auxiliary tool (51) for turning the threaded spindle.

10. Positioning system according to claims 8 and 9, **characterized in that** the receptacles (42, 52) are positioned coaxially with respect to each other and the receptacle (52) for receiving the accessory tool (51) for turning the threaded spindle (15) is accessible through the receptacle (42) for receiving the accessory tool (41) for pivoting the ball (9).

11. Positioning system according to one of the preceding claims, **characterized in that** one single actuator means (31, 76, 85, 115, 123) is provided for actuating the clamp mechanism.

12. Positioning system according to one of the preceding claims, **characterized in that** the clamp mechanism is formed as a wedge clamp device.

13. Positioning system according to claims 11 and 12, **characterized in that** the wedge clamp device includes a pressure dome (72) which slides on an oblique plane (71) and which is adapted to be pressed against the ball (9) by tightening a locking screw (31) forming the actuator means.

14. Positioning system according to one of claims 1 to 11, **characterized in that** the clamp mechanism is formed as a ring clamp device.

15. Positioning system according to claims 11 and 14, **characterized in that** the ring clamp device includes a clamp ring (76) forming the actuator means and being adapted to be screwed into a thread (77) of the socket to press against the ball (9).

16. Positioning system according to claims 1 to 11, **characterized in that** the clamp mechanism is formed as a lever clamp device.

17. Positioning system according to claims 11 and 16, **characterized in that** the lever clamp device includes a pressure dome (78) which acts as a lever and is adapted to be swivelled around a lever thrust bearing (79) to press against the ball (9) by tightening a locking screw (31) forming the actuator means.

18. Positioning system according to one of claims 1 to 11, **characterized in that** the clamp mechanism is formed as a cam clamp device.

19. Positioning system according to claims 11 and 18, **characterized in that** the cam clamp device includes a pressure dome (82, 93) cooperating with a cam disc (83, 91) torsionally attached to a locking screw (85) forming the actuator means, the cam disc being adapted to press said pressure dome against the ball (9) when the locking screw and hence the cam disk (83, 91) is turned.

20. Positioning system according to one of claims 1 to 11, **characterized in that** the clamp mechanism is formed as a clip-type clamp device.

21. Positioning system according to claims 11 and 20, **characterized in that** the socket (8) is slotted in a way to elastically surround the ball (9) in a manner that the socket is pressed against the ball by tightening a locking-screw (31) forming the actuator means.

22. Positioning system according to one of claims 1 to 11, **characterized in that** the clamp mechanism is formed as formed as a thrust pin clamp device.

23. Positioning system according to claims 11 and 21, **characterized in that** a threaded thrust pin (115) forming the actuator means is screwed into a threaded hole (116) of the holding device (2) and is adapted to press against the ball (9) by being turned.

24. Positioning system according to claims 11 and 22, **characterized in that** a thrust pin (120, 131) forming the actuator means is adapted to press against the ball (9) by swivelling a cam (123).

25. Positioning system according to claim 24, **characterized in that** the thrust pin (131) is elastically pretensioned in direction towards the ball (9).

26. Positioning system according to one of the preceding claims, **characterized in that** the actuator means (31, 85) provided with a loss preventor (36, 80, 89).

27. Positioning system according to one of the preceding claims, **characterized in that** the accessory tool (51) for turning the threaded spindle (15) has a spherical head (99) with a polygonal profile that is adapted to positively engage the receptacle (52) of the threaded spindle (15), the receptacle having a complementary polygonal profile.

28. Positioning system according to one of the preceding claims, **characterized in that** it is made of implantable metals like e.g. pure titanium, its implantable alloys as well as implantable steels.

29. Positioning system according to one of the preceding claims, **characterized in that** the actuator or sensor means (54) is construed for diagnosis, therapy and/or surgical applications for the temporary or permanent implantation of said means.

30. Positioning system according to one of the preceding claims, **characterized in that** the actuator means (54) is an implantable electromechanical hearing aid converter.

31. Positioning system according to one of the preceding claims, **characterized in that** it is construed for positioning and coupling an attached hearing aid converter (54) to any arbitrary point of the middle ear including any arbitrary coupling point to the inner ear, such as a natural or artificial window, as a body-fixed target point (58) and for fixing the hearing aid converter in this position.

32. Positioning system according to claim 31, **characterized in that** it is construed for positioning and coupling an attached hearing aid converter (54) to the hammer (104), anvil (105) or stirrup (106) as a body-fix target point (58) and for fixing the hearing aid converter in this position.

33. Positioning system according to claim 31 or 32, **characterized in that** the hearing aid converter (54) is construed as an actuator component of a completely or partially implantable hearing aid system.

34. Positioning system according to one of the preceding claims, **characterized in that** the positioning system (1) is construed for altogether one axial (57) and three rotatory (38, 39, 40) movements for the actuator or sensor means (54) and its free actuator end (56) in body orifices of the human body as a result of the degrees of freedom of the ball-and-socket joint (7) and the arrangement consisting of spindle nut (46) and threaded spindle (15).

35. Positioning system according to one of the preceding claims, characterized that it is construed, with the clamping of the ball-and-socket joint (7) being released, for adjustment of all three rotatory degrees of freedom (38, 39, 40) of the ball-and-socket joint by means of the accessory tool (41) adapted to engage the ball (9).

36. Positioning system according to one of claims 30 or 31, **characterized in that** the instantaneous position of the three rotatory degrees of freedom (38, 39, 40) is maintained by frictional forces when the clamp mechanism (28) of the ball-and-socket joint (7) is released and is maintained permanently after closing the clamp mechanism.

37. Positioning system according to one of the preceding claims, **characterized in that** the actuator elements (31, 41, 51, 77, 85) for manual positioning of the ball-and-socket joint (7) and the carriage (47) as well as for clamping the ball-and-socket joint are pointing away from the body of the patient towards the surgeon.

38. Positioning system according to one of the preceding claims, **characterized in that** the construction and the geometrical dimensions of the positioning system (1) are designed in a way that the operating surgeon has free sight onto at least the free actuator end (56) of the actuator or sensor means (54) as well as onto the area of implantation including the target point (58) in the body of the patient during working with the naked eye as well as during the usage of a microscope.

## Revendications

1. Système de positionnement (1) pouvant être implanté et fixé de façon permanente pour la liaison solidaire sans jeu avec un os du corps humain, notamment l'os crânien, comportant :
- une articulation à rotule (7) avec une bille d'articulation (9) et un logement de bille (8), dont la bille d'articulation peut être pivotée manuellement par rapport au logement de bille au moyen d'un outil auxiliaire (41), et bloquée par un mécanisme de serrage (28),
- un rail de guidage (14) droit relié solidairement à la bille d'articulation (9) de l'articulation à rotule (7),
- une broche filetée (15) à filetage extérieur, qui est montée en rotation par rapport au rail de guidage (14), mais de façon immobile dans la direction axiale,
- un coulisseau (47), comportant un écrou de broche (46) avec un taraudage qui est en prise avec le filetage extérieur de la broche filetée (15), et qui peut être positionné librement le long du rail de guidage (14) entre des butées d'extrémité (19, 45) par une rotation manuelle de la broche filetée (15) au moyen d'un outil auxiliaire (51),
- une réception (53) prévue sur le coulisseau (47) pour un moyen actionneur ou sensoriel (54) à positionner ou à fixer, et
- un élément de retenue (2) pouvant être vissé sur l'os, auquel sont fixés le logement de bille (8) et le mécanisme de serrage (28) de l'articulation à rotule (7),
**caractérisé en ce que**
le rail de guidage (14) comporte des surfaces extérieures de guidage (60, 61), qui sont maintenues en prise de glissement avec des surfaces intérieures de guidage (62, 63) du coulisseau (47), et **en ce que** les surfaces intérieures de guidage (62, 63) sont formées par des flancs (64, 65) du coulisseau (47), qui sont disposés élastiquement par rapport à l'écrou de broche (46) dans un plan situé perpendiculairement à l'axe longitudinal de la broche filetée (15).

2. Système de positionnement selon la revendication 1, **caractérisé en ce qu'**à l'état hors contrainte, les surfaces intérieures de guidage (62, 63) sont situées à un écartement mutuel qui est inférieur d'une cote prédéterminée à l'écartement mutuel entre les surfaces extérieures de guidage (60, 61).

3. Système de positionnement selon la revendication 1 ou 2, **caractérisé en ce que** les flancs (64, 65) sont formés sur des bras élastiques (66, 67), qui sont reliés d'un seul tenant à un corps (68) de coulisseau comportant l'écrou de broche (46).

4. Système de positionnement selon l'une quelconque des revendications précédentes, **caractérisé en ce que** les surfaces intérieures de guidage (62, 63) du coulisseau (47), et les surfaces extérieures de guidage (60, 61) du rail de guidage (14), sont respectivement situées diamétralement à l'opposé les unes des autres, et sont disposées symétriquement par rapport à un plan de symétrie longitudinal (69) du coulisseau contenant l'axe longitudinal de l'écrou de broche (46), ou symétriquement par rapport à un plan de symétrie longitudinal contenant l'axe longitudinal de la broche filetée (15) du module constitué du rail de guidage et de la broche filetée.

5. Système de positionnement selon la revendication 4, **caractérisé en ce que** les surfaces intérieures de guidage (62, 63) du coulisseau (47), et les surfaces extérieures de guidage (60, 61) du rail de guidage (14), forment respectivement avec le plan de symétrie longitudinal (69) du coulisseau, ou avec le plan de symétrie longitudinal du module constitué du rail de guidage et de la broche filetée (15), un angle se situant dans la plage de 10° à 60°.

6. Système de positionnement selon l'une quelconque des revendications précédentes, **caractérisé en ce que** l'écrou de broche (46) et la broche filetée (15) sont munis d'un filetage autobloquant.

7. Système de positionnement selon l'une quelconque des revendications précédentes, **caractérisé en ce que**, au niveau de son extrémité située du côté de la bille, et au niveau de son extrémité opposée à la bille d'articulation (9), la broche filetée (15) est montée en rotation dans le dispositif constitué de la bille d'articulation et du rail de guidage (14).

8. Système de positionnement selon l'une quelconque des revendications précédentes, **caractérisé en ce que** la bille d'articulation (9) comporte une réception (42) pour l'application de l'outil auxiliaire (41) servant à pivoter la bille d'articulation.

9. Système de positionnement selon l'une quelconque des revendications précédentes, **caractérisé en ce que** la broche filetée (15) comporte sur son extrémité orientée vers la bille d'articulation (9) une réception (52) pour l'application de l'outil auxiliaire (51) servant à tourner la broche filetée.

10. Système de positionnement selon les revendications 8 et 9, **caractérisé en ce que** les réceptions (42, 52) sont disposées coaxialement l'une par rapport à l'autre, et **en ce que** la réception (52) pour l'application de l'outil auxiliaire (51) servant à tourner la broche filetée (15) est accessible par la réception (42) pour l'application de l'outil auxiliaire (41) servant à pivoter la bille d'articulation (9).

11. Système de positionnement selon l'une quelconque des revendications précédentes, **caractérisé en ce qu'**un seul élément de réglage (31, 76, 85, 115, 123) est prévu pour l'actionnement du mécanisme de serrage.

12. Système de positionnement selon l'une quelconque des revendications précédentes, **caractérisé en ce que** le mécanisme de serrage est agencé sous la forme d'un dispositif de serrage à clavette.

13. Système de positionnement selon les revendications 11 et 12, **caractérisé en ce que** le dispositif de serrage à clavette comporte une calotte de pression (72) coulissant sur un plan incliné (71), qui peut être pressée contre la bille d'articulation (9) par le serrage d'une vis de serrage (31) constituant l'élément de réglage.

14. Système de positionnement selon l'une quelconque des revendications 1 à 11, **caractérisé en ce que** le mécanisme de serrage est agencé sous la forme d'un dispositif de serrage à bague.

15. Système de positionnement selon les revendications 11 et 14, **caractérisé en ce que** le dispositif de serrage à bague comporte une bague de serrage (76) pouvant être vissée dans un filetage (77) du logement de bille, et pressée contre la bille d'articulation (9), laquelle bague constitue l'élément de réglage.

16. Système de positionnement selon l'une quelconque des revendications 1 à 11, **caractérisé en ce que** le mécanisme de serrage est agencé sous la forme d'un dispositif de serrage à levier.

17. Système de positionnement selon les revendications 11 et 16, **caractérisé en ce que** le dispositif de serrage à levier comporte une calotte de pression (78) à effet de levier qui, par le serrage d'une vis de serrage (31) constituant l'élément de réglage, peut être pivotée autour d'une butée de levier (79) et pressée contre la bille d'articulation (9).

18. Système de positionnement selon l'une quelconque des revendications 1 à 11, **caractérisé en ce que** le mécanisme de serrage est agencé sous la forme d'un dispositif de serrage à excentrique.

19. Système de positionnement selon les revendications 11 et 18, **caractérisé en ce que** le dispositif de serrage à excentrique comporte une calotte de pression (82, 93), qui agit conjointement avec un disque d'excentrique (83, 91), lequel est de son côté relié solidairement en rotation à une vis de serrage (85) constituant l'élément de réglage, et **en ce que** la calotte de pression (82, 93) peut être pressée contre la bille d'articulation (9) par la rotation de la vis de serrage (85), et par conséquent du disque d'excentrique (83, 91).

20. Système de positionnement selon l'une quelconque des revendications 1 à 11, **caractérisé en ce que** le mécanisme de serrage est agencé sous la forme d'un dispositif de serrage à collier.

21. Système de positionnement selon les revendications 11 et 20, **caractérisé en ce que** le logement de bille (8) est fendu de telle sorte qu'il entoure élastiquement la bille d'articulation (9), et **en ce que** le logement de bille peut être pressé contre la bille d'articulation par le serrage d'une vis de serrage (31) constituant l'élément de réglage.

22. Système de positionnement selon l'une quelconque des revendications 1 à 11, **caractérisé en ce que** le mécanisme de serrage est agencé sous la forme d'un dispositif de serrage à tige de pression.

23. Système de positionnement selon les revendications 11 et 22, **caractérisé en ce qu'**une tige de pression filetée (115) constituant l'élément de réglage peut être vissée dans un alésage taraudé (116) de l'élément de retenue (2), et pressée par rotation contre la bille d'articulation (9).

24. Système de positionnement selon les revendications 11 et 22, **caractérisé en ce qu'**une tige de pression (120, 131) constituant l'élément de réglage peut être pressée contre la bille d'articulation (9) par le pivotement d'un excentrique (123).

25. Système de positionnement selon la revendication 24, **caractérisé en ce que** la tige de pression (131) est précontrainte élastiquement en direction de la bille d'articulation (9).

26. Système de positionnement selon l'une quelconque des revendications précédentes, **caractérisé en ce que** l'élément de réglage (31, 85) est muni d'une protection anti-perte (36, 80, 89).

27. Système de positionnement selon l'une quelconque des revendications précédentes, **caractérisé en ce que** l'outil auxiliaire (51) servant à tourner la broche filetée (15) possède une tête sphérique (99) à profil polygonal, qui peut être engagée par complémentarité de formes dans un évidement (52) à profil polygonal complémentaire de la broche filetée (15).

28. Système de positionnement selon l'une quelconque des revendications précédentes, **caractérisé en ce qu'**il est réalisé en métaux implantables, tels que par exemple en titane pur, ses alliages susceptibles d'être implantés, ainsi qu'en aciers spéciaux pour implants.

29. Système de positionnement selon l'une quelconque des revendications précédentes, **caractérisé en ce que** les moyens actionneurs ou sensoriels (54) destinés au diagnostic, à la thérapie et/ou à des applications chirurgicales, sont conçus pour l'implantation temporaire ou permanente desdits moyens.

30. Système de positionnement selon l'une quelconque des revendications précédentes, **caractérisé en ce qu'**en ce qui concerne le moyen actionneur (54), il s'agit d'un convertisseur électromécanique de prothèse auditive implantable.

31. Système de positionnement selon l'une quelconque des revendications précédentes, **caractérisé en ce qu'**il est conçu pour le positionnement et l'accouplement d'un convertisseur (54) de prothèse auditive, qui y est fixé, au niveau d'un point quelconque de l'oreille moyenne, y compris de n'importe quel point d'accouplement de l'oreille interne, tel qu'une fenêtre naturelle ou artificielle, en tant que point cible (58) fixe du corps, et pour la fixation du convertisseur de prothèse auditive dans cette position.

32. Système de positionnement selon la revendication 31, **caractérisé en ce qu'**il est conçu pour le positionnement et l'accouplement d'un convertisseur (54) de prothèse auditive, qui y est fixé, au niveau du marteau (104), de l'enclume (105) ou de l'étrier (106) en tant que point cible (58) solidaire du corps, et pour la fixation du convertisseur de prothèse auditive à cette position.

33. Système de positionnement selon la revendication 31 ou 32, **caractérisé en ce que** le convertisseur (54) de prothèse auditive est prévu en tant que composant actionneur d'une prothèse auditive partiellement ou entièrement implantable.

34. Système de positionnement selon l'une quelconque des revendications précédentes, **caractérisé en ce que**, en raison des degrés de liberté de l'articulation à rotule (7) et du dispositif constitué de l'écrou de broche (46) et de la broche filetée (15), le système de positionnement (1) est en résumé conçu pour un mouvement axial (57) et trois mouvements de rotation (38, 39, 40) du moyen actionneur ou sensoriel (54), et de son extrémité active libre (56), dans des ouvertures corporelles du corps humain.

35. Système de positionnement selon l'une quelconque des revendications précédentes, **caractérisé en ce qu'**à l'état dissocié du serrage de l'articulation à rotule (7), il est conçu pour un réglage de tous les trois degrés de liberté de rotation (38, 39, 40) de l'articulation à rotule au moyen de l'outil auxiliaire (41) pouvant être mis en prise avec la bille d'articulation (9).

36. Système de positionnement selon les revendications 30 et 31, **caractérisé en ce qu'**à l'état dissocié du mécanisme de serrage (28) de l'articulation à rotule (7), la position momentanée des trois degrés de liberté de rotation (38, 39, 40) est assurée par des forces de friction, et est maintenue de façon permanente après la fermeture du mécanisme de serrage.

37. Système de positionnement selon l'une quelconque des revendications précédentes, **caractérisé en ce que** les éléments de commande (31, 41, 51, 77, 85) destinés au positionnement manuel de l'articulation à rotule (7) et du coulisseau (47), ainsi qu'au serrage de l'articulation à rotule, sont écartés du corps du patient et orientés vers l'opérateur.

38. Système de positionnement selon l'une quelconque des revendications précédentes, **caractérisé en ce que** la construction et les dimensions géométriques du système de positionnement (1) sont configurées de telle sorte que l'opérateur ait une libre vue, aussi bien en travaillant à l'oeil nu qu'en utilisant un microscope, au moins sur l'extrémité active libre (56) du moyen actionneur ou sensoriel (54), ainsi que sur la zone d'implantation avec le point cible (58) dans le corps du patient.
